# EUROPÄISCHE PATENTSCHRIFT

(11) **EP 0 644 750 B1**
(45) Veröffentlichungstag und Bekanntmachung des Hinweises auf die Patenterteilung: **10.04.1996**
(21) Anmeldenummer: 93912872.4
(22) Anmeldetag: 03.06.1993
(51) Int. Cl.: A61K 7/06

(54) **HAARBEHANDLUNGSMITTEL**
HAIR TREATMENT AGENT
AGENT FIXANT POUR CHEVEUX

(30) Priorität: 11.06.1992 DE 4219065
(43) Veröffentlichungstag der Anmeldung: 29.03.1995
(73) Patentinhaber: Henkel Kommanditgesellschaft auf Aktien, D-40191 Düsseldorf (DE)
(72) Erfinder: WÜLKNITZ, Peter, D-4018 Langenfield (DE); SCHOLTEN, Andreas, D-4060 Viersen 1 (DE); NIEMANN, Annedore, D-4010 Hilden (DE)
(86) Internationale Anmeldenummer: EP9301400
(87) Internationale Veröffentlichungsnummer: WO9325178

(56) Entgegenhaltungen:
- WO-A-90/12559
- WO-A-90/14072
- DE-A- 4 132 776
- FR-A- 2 424 738
- GB-A- 1 595 649

## Beschreibung

Gegenstand der Erfindung sind Zubereitungen zum Festigen von Haaren auf Basis von Ethanol, die frei von fluorchlorkohlenwasserstoffhaltigen Treibmitteln und Parfümölen sind und als Filmbildner spezielle Polymere enthalten.

Eine ansprechend aussehende Frisur wird heute allgemein als unverzichtbarer Teil eines gepflegten Äußeren angesehen. Dabei gelten aufgrund von aktuellen Modeströmungen immer wieder Frisuren als chic, die sich bei vielen Haartypen nur unter Verwendung bestimmter Haarfestlegemittel aufbauen bzw. für einen längeren Zeitraum aufrechterhalten lassen. Solche Haarfestlegemittel werden üblicherweise in Form von Haarsprays bereitgestellt und enthalten als zwingende Komponenten in der Regel Lösungsmittel, Filmbildner, Treibmittel und Parfümöle.

Obwohl diese Rezepturen im Vergleich zu anderen Kosmetika und Körperreinigungsmitteln auf den ersten Blick einfach aufgebaut erscheinen, so sind sie doch in jüngster Zeit aus einer Reihe von Gründen Gegenstand intensiver Entwicklungstätigkeit.

Wurden früher Fluorchlorkohlenwasserstoffe als ideale, inerte Treibmittel angesehen, so führte die aktuelle Ozon-Diskussion zu einem weitestgehenden Ersatz dieser Treibmittel. Heute werden in der Regel als Treibmittel niedere Alkane wie Propan/Butan oder Dimethylether verwendet. Deren Verwendung erfordert entweder einen insbesondere beim Abfüllen deutlich größeren technischen Aufwand oder ist mit anderen Nachteilen, z.B. leichter Brennbarkeit, behaftet.

Dies führte einerseits zu einer Renaissance der Pumpsprays, bei denen treibmittelfreie Lösungen mittels einer mechanischen Vorrichtung versprüht werden. Hier bedarf es nicht unwesentlicher technischer Anstrengungen, um ein Sprühverhalten zu erhalten, das dem der FCKW-haltigen Sprays nahekommt.

Zum anderen wird ein Versprühen der Formulierungen mit bekannten, wohlfeilen Gasen wie CO₂, Stickstoff oder Luft angestrebt.

Bei den treibmittelfreien Formulierungen tritt bei Mischungen auf Basis Ethanol ein zusätzliches Problem auf. Aufgrund des hohen Alkoholgehaltes ist auch der Anteil des Vergällungsmittels deutlich höher als in treibmittelhaltigen Formulierungen. Ausgerechnet das übliche Vergällungsmittel Diethylphthalat, das dann in Konzentrationen von mehr als 0,5 Gew.-%, bezogen auf die gesamte Zubereitung, vorliegt, wirkt aber als Weichmacher für als Filmbildner verwendete Polymeren, wodurch deren haarfestigende Eigenschaften stark herabgesetzt werden.

Weiterhin werden vom Konsumenten immer häufiger parfümfreie Formulierungen gewünscht, um möglichen Risiken hinsichtlich allergischer Reaktionen vorzubeugen. Es besteht daher auch ein Bedarf an Formulierungen, die so geruchsneutral sind, daß sie auch parfümfrei vom Konsumenten akzeptiert werden.

Es wurde nun gefunden, daß sich fluorchlorkohlenwasserstofffreie Formulierungen mit guten Eigenschaften zum Festigen von Haaren auf Basis von Ethanol herstellen lassen, wenn bestimmte Polymere als Filmbildner eingesetzt werden. Diese Zubereitungen sind gewünschtenfalls auch gänzlich treibmittelfrei formulierbar. In diesem Fall kann der Verbraucher die Verpackung, z. B. einen Pumpspray-Behälter, auch öffnen und die Zubereitung nachträglich mit einem Parfümöl seiner Wahl versehen.

Gegenstand der Erfindung ist daher eine Zubereitung zum Festigen von Haaren auf Basis einer alkoholischen oder wäßrig-alkoholischen Lösung, enthaltend Filmbildner und Lösungsmittel sowie übliche kosmetische Bestandteile, dadurch gekennzeichnet, daß
- die Zubereitung zu mindestens 40 Gew.-% aus Ethanol besteht,
- frei ist von fluorchlorkohlenwasserstoffhaltigen Treibmitteln und Parfümölen und
- der Filmbildner ein anionisches, amphoteres oder zwitterionisches Polymeres oder Copolymeres ist, das als Monomeres eine ganz oder teilweise neutralisierte, ethylenisch ungesättigte Carbonsäure mit 3 oder 4 Kohlenstoffatomen oder eine ganz oder teilweise neutralisierte, ethylenisch ungesättigte Dicarbonsäure mit 4 Kohlenstoffatomen oder deren Monoester mit einem C₁₋₄-Alkohol enthält und frei ist von Vinylpyrrolidon Gruppen und die Zubereitung Phtalsäureester in einer solchen Menge enthält, daß das Verhältnis der Massen von Filmbildner und Phtalsäureester 3:1 bis 10:1 beträgt.

Die erfindungsgemäßen Zubereitungen sind auf Basis niederer Alkohole formuliert.

Ethanol ist als Lösungmittel zwingend in einer Menge von mindestens 40 Gew.-%, bezogen auf die gesamte Zubereitung, enthalten. Neben Ethanol können die Zubereitungen weitere niedere Alkohole, beispielsweise n-Propanol und Isopropanol, als Lösungsmittel enthalten. Bevorzugt enthalten die erfindungsgemäßen Zubereitungen zwischen 50 und 95 Gew.-% an niederen Alkoholen.

Weiterhin können die Formulierungen als Lösungsmittel Wasser in Mengen bis zu 20 Gew.-% enthalten. Lineare und verzweigte Alkane mit 5 bis 7 C-Atomen und Ketone mit 3 bis 6 C-Atomen können ebenfalls als Lösungsmittel in Mengen von bevorzugt weniger als 20 Gew.-% in den erfindungsgemäßen Zubereitungen enthalten sein.

Durch geeignete Wahl der Lösungsmittel ist es möglich, die Zubereitungen als klare Lösungen zu formulieren. Solche klare Lösungen werden vom Verbraucher üblicherweise aus ästhetischen Gründen bevorzugt. Sofern die erfindungsgemäßen Mittel daher in durchsichtigen Behältern formuliert werden, sind daher Zubereitungen in Form klarer Lösungen bevorzugt.

Die zur erfindungsgemäßen Verwendung als Filmbildner geeigneten Polymeren werden in ihren Eigenschaften durch andere Komponenten, insbesondere das für Ethanol übliche Vergällungsmittel Diethylphthalat, nicht negativ beeinflußt.

Dies ist bei solchen Polymeren der Fall, die als Monomeres mindestens eine ganz oder teilweise neutralisierte, ethylenisch ungesättigte Carbonsäure mit 3 oder 4 Kohlenstoffatomen enthalten. Geeignete ethylenisch ungesättigte Carbonsäuren sind Acrylsäure, Crotonsäure und Methacrylsäure.

Weiterhin sind solche Polymeren geeignet, die als Monomeres mindestens eine ethylenisch ungesättigte Dicarbonsäure mit 4 C-Atomen enthalten, also Fumarsäure oder Maleinsäure, enthalten. Maleinsäure ist dabei bevorzugt. Erfindungsgemäß ebenfalls geeignet sind die Monoester dieser Dicarbonsäuren mit Alkoholen mit 1 bis 4 Kohlenstoffatomen. Butylmaleat ist ein besonders bevorzugtes Monomeres.

Die Carbonsäuregruppen der Polymeren sind ganz oder teilweise mit Basen neutralisiert. Der Neutralisationsgrad liegt üblicherweise zwischen 60 und 100 %. Polymere, bei denen nur ein kleiner Teil der Carbonsäuregruppen nicht neutralisiert ist, weisen in den erfindungsgemäßen Zubereitungen besonders gute Eigenschaften auf. Polymere, die einen Neutralisationsgrad zwischen 80 und 95 % aufweisen, sind daher bevorzugt.

Zur Neutralisation der Carbonsäuregruppen können organische oder anorganische Basen verwendet werden. Geeignete anorganische Basen sind beispielsweise Alkalimetallhydroxide wie Natriumhydroxid und Kaliumhydroxid, Erdalkalimetallhydroxide wie Calciumhydroxid und Magnesiumhydroxid, Aluminiumhydroxid und Ammoniumhydroxid. Geeignete organische Basen sind primäre, sekundäre und tertiäre Amine und Alkanolamine mit 1 bis 4 Kohlenstoffatomen in den Alkyl- bzw. Hydroxyalkylgruppen. Bevorzugt werden organische Basen zur Neutralisation verwendet. Triethanolamin und 2-Amino-2-methyl-1-propanol sind besonders bevorzugte Basen.

Um sicherzustellen, daß die Polymeren die gewünschten Eigenschaften aufweisen, dürfen sie keine Vinylpyrrolidon-Monomere enthalten. Nur Polymere ohne diese Monomer-Komponente werden in ihren Eigenschaften auf dem Haar auch durch hohe Diethylphthalatgehalte in der Lösung nicht negativ beeinflußt.

Je nach Art der gewählten Co-Monomeren können die Polymeren anionisch, zwitterionisch oder amphoter sein.

Erfindungsgemäß geeignete anionische Polymere sind:
- Vinylacetat/Crotonsäure-Copolymere, wie sie beispielsweise unter den Bezeichnungen Resyn^{R} (National Starch), Luviset^{R} (BASF) und Gafset^{R} (GAF) im Handel sind.
- Acrylsäure/Ethylacrylat/N-tert.-Butylacrylamid-Terpolymere, wie sie beispielsweise als Ultrahold^{R}8 im Handel sind.
- Vinylacetat/Mono-n-butylmaleat/Isobornylacrylat-Copolymere, wie sie unter dem Warenzeichen Advantage^{R} (GAF) erhältlich sind. Advantage^{R} CP ist ein bevorzugtes Polymeres.

Geeignete zwitterionische Polymere sind beispielsweise die in den deutschen Patentanmeldungen DE 39 29 973, DE 21 50 557, DE 28 17 369 und DE 37 08 451 offenbarten Polymerisate. Acrylamidopropyltrimethylammoniumchlorid/Acrylsäure-Copolymerisate und deren Alkali- und Ammoniumsalze sind besonders bevorzugte zwitterionische Polymere.

Geeignete amphomere Polymere sind beispielsweise Octylacrylamid/Methylmethacrylat/tert.Butylaminoethylmethacrylat/2-Hydroxypropylemethacrylat-Copolymere.

Bevorzugt wird als Filmbildner ein anionisches Polymeres verwendet. Luviset CA-66 und Ultrahold^{R}8 sind ganz besonders bevorzugte Polymere.

Die erfindungsgemäßen Zubereitungen enthalten die polymeren Filmbildner bevorzugt in Mengen von 1 bis 10 Gew.-% , bezogen auf die gesamte Zubereitung. Mengen von 2 bis 7 Gew.-% sind besonders bevorzugt.

Die erfindungsgemäßen Zubereitungen sind frei von fluorchlorkohlenwasserstoffhaltigen Treibmitteln. Es ist bevorzugt, die Zubereitungen treibmittelfrei als sogenannte Pumpsprays zu formulieren.

Gleichwohl können diese Zubereitungen auch als treibmittelhaltige Mittel formuliert werden. Als Treibmittel geeignet sind insbesondere Luft, Stickstoff und Edelgase. Erfindungsgemäße Zubereitungen enthalten diese Treibmittel bevorzugt in Mengen von 0,5 - 2,0 Gew.-%, bezogen auf die gesamte Zubereitung.

Die erfindungsgemäßen Zubereitungen sind wegen des geringen Eigengeruchs der Bestandteile, insbesondere der verwendeten Polymeren, hervorragend dazu geeignet, parfümfrei eingesetzt zu werden.

Die erfindungsgemäßen Zubereitungen sind daher weiterhin frei von Parfümölen. Unter Parfümölen sind im Sinne der Erfindung sowohl Mischungen von Riechstoffen, die dem Fachmann bekannte Anbieter zum Zwecke der Parfümierung von Kosmetika, Wasch- und Reinigungsmitteln bereitstellen, als auch natürliche oder synthetische Einzelkomponenten oder Mischungen, die als Riechstoffe verwendet werden, zu verstehen.

Eine weitere, interessante Ausführungsform der Erfindung besteht darin parfümfreie Formulierungen für sogenannte Pumpsprays bereitzustellen. Bei solchen Sprays lassen sich die nicht unter Druck stehenden Behälter leicht öffnen. Der Konsument wird dadurch in die Lage versetzt, die parfümfrei erworbene Formulierung mit einem Parfüm seiner Wahl zu versetzen und die Duftnote des Kosmetikums so selbst zu bestimmen. Beispiele für mögliche Parfümierungen sind Eau de Toilette, Eau de Parfum sowie Parfümöle der Duftrichtungen frisch/citrus("grün"), blumig, aldehydisch, chypre und "orientalisch". Die entsprechenden Parfümöle sind dem Fachmann geläufig.

Weiterhin können die erfindungsgemäßen Zubereitungen alle für solche Mittel üblichen kosmetischen Inhaltsstoffe enthalten. Diese weiteren Inhaltsstoffe sind üblicherweise nur in untergeordneten Mengen, d.h. in der Regel in Mengen von bis zu einigen Gew.-%, enthalten.

Übliche Bestandteile sind:
- Strukturanten wie Glucose und Maleinsäure,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate,
- Dimethylisosorbid und Cyclodextrine,
- Farbstoffe,
- Wirkstoffe wie Panthenol, Allantoin, Pyrrolidoncarbonsäuren, Pflanzenextrakte und Vitamine,
- Lichtschutzmittel,
- Konsistenzgeber wie Anlagerungsprodukte von 5 bis 60 Mol Ethylenoxid an Rizinusöl und gehärtetes Rizinusöl,
- Nichtionogene Polymere wie Polyvinylpyrrolidon, Copolymere des Vinylpyrrolidons z. B. mit Vinylacetat und Polysiloxane.
- Kationische Polymere wie beispielsweise quaternisierte Celluloseether, Polysiloxane mit quaternären Gruppen, Acrylamid-Dimethyldiallylammoniumchlorid-Copolymere sowie mit Diethylsulfat quaternierte Dimethylaminomethacrylat-Vinylpyrrolidon-Copolymere und
- Silikonöle.

Die erfindungsgemäßen Zubereitungen können weiterhin gewünschtenfalls geringe Mengen an Konservierungsmitteln enthalten. Es wurde aber festgestellt, daß die erfindungsgemäßen Zubereitungen auch ohne weitere Konservierungsstoffe in aller Regel eine ausreichende Stabilität gegen Keimbefall aufweisen. Konservierungsmittelfreie Formulierungen sind daher im Rahmen der erfindungsgemäßen Lehre bevorzugt.

Die erfindungsgemäße Lehre, daß bestimmte Polymeren in ihren Eigenschaften auf dem Haar nicht durch im Ethanol vorhandene Vergällungsmittel mit Weichmachereigenschaften negativ beeinflußt werden, läßt sich aber auch gezielt zur Verbesserung von Polymereigenschaften einsetzen. Einige, an sich für die Haarpflege gut geeignete Polymere weisen den Nachteil einer etwas zu hohen Sprödigkeit auf dem Haar auf. Diese Sprödigkeit wird durch die im Ethanol vorhandenen Vergällungsmittel mit Weichmachereigenschaften zwar weitgehend, aber noch nicht optimal abgebaut. Dieses Optimum kann aber durch Zusatz weiterer Weichmacher erreicht werden. Vorteilhafterweise wird man eine dem Vergällungsmittel strukturell und chemisch ähnliche Verbindung verwenden. Bevorzugt werden daher Phthalsäuremono- und -diester mit niederen Alkoholen verwendet. Beispiele für solche Ester sind Phthalsäuremono- und - dimethylester sowie Phthalsäuremono- und -diethylester. Bevorzugt werden Filmbildner und Phthalsäureester in Massenverhältnis 3:1 bis 10:1 eingesetzt. Die Verwendung von Phthalsäurediethylester ist besonders bevorzugt.

Gegenstand der Erfindung ist ebenfalls ein Verfahren zur Behandlung, insbesondere zum Festigen von Haaren, dadurch gekennzeichnet, daß eine Zubereitung nach einem der Ansprüche 1 bis 10 verwendet wird.

Ebenfalls Gegenstand der Erfindung ist ein Verfahren zur Behandlung der Haare, bei dem eine Zubereitung nach einem der Ansprüche 1 bis 10 verwendet wird, der nachträglich, insbesondere unmittelbar vor der Verwendung, ein Parfümöl zugesetzt wurde.

Die folgenden Beispiele sollen den Gegenstand der Erfindung weiter erläutern.

### Beispiele

Es wurden folgende Formulierungen hergestellt (Angaben in Gewichtsteilen):

| Komponente | Beispiel 1 | Beispiel 2 | Vergleich |
|---|---|---|---|
| Ethanol abs. | 76,0¹ | 86,2² | 86,2² |
| Wasser | 8,55 | 8,3 | 8,30 |
| Ultrahold^{R}8³ | 5,0 | - | - |
| Luviset^{R}CA 66⁴ | - | 5,0 | - |
| Luviflex^{R}VBM 35⁵ | - | - | 5,0 |
| 2-Amino-2-methyl-1-propanol | 0,45⁶ | 0,45⁷ | 0,45⁶ |
| Isopropanol | 10,0 | - | - |
| Parfümöl | - | 0,05 | 0,05 |

| | | | |
|---|---|---|---|
| ¹ davon 1,0 % Vergällungsmittel Diethylphthalat | | | |
| ² davon 1,2 % Vergällungsmittel Diethylphthalat | | | |
| ³ Terpolymer aus Acrylsäure, Ethylacrylat und N-tert.Butyl-acrylamid (BASF) | | | |
| ⁴ Copolymerisat aus Vinylacetat und Crotonsäure im Verhältnis 90:10 (BASF) | | | |
| ⁵ Copolymerisat auf Basis von Vinylpyrrolidon, tert.Butylacrylat und Methacrylsäure (50 % in Ethanol) (BASF) | | | |
| ⁶ ergibt für das Polymere einen Neutralisationsgrad von 90 % | | | |
| ⁷ ergibt für das Polymere einen Neutralisationsgrad von 80 % | | | |

Beim Versprühen mit einem handelsüblichen Spraysystem "Airspray" (B.V. Braune, Aerosol Report 29 (12), 612 (1990)) wurden folgende Ergebnisse erzielt:

| Komponente | Beispiel 1 | Beispiel 2 | Vergleich |
|---|---|---|---|
| Festigung des Haares | sehr gut | gut | schwach |
| Klebrigkeit des Filmes | gering | gering | stark |
| Eigengeruch der parfümfreien Zubereitung | neutral | neutral | stark |
| geruchliche Verträglichkeit mit Parfümölen des Typs | | | |
| - "grün" | gut | gut | schlecht |
| - blumig | befriedigend | gut | schlecht |
| - aldehydisch | gut | gut | befriedigend |
| - chypre | gut | gut | schlecht |
| - "orientalisch" | gut | gut | befriedigend |

| Komponente | Beispiel 3 | Beispiel 4 | Beispiel 5 | Beispiel 6 |
|---|---|---|---|---|
| Ethanol abs. | 75,0¹ | 66,0⁸ | 84,55² | 76,0² |
| Wasser | 8,5 | 8,55 | - | 8,45 |
| n-Heptan | - | 10,0 | - | - |
| Ultrahold^{R}8 | 5,0 | 5,0 | 5,0 | 5,0 |
| 2-Amino-2-methyl-1-propanol | 0,45 | 0,45 | 0,45 | 0,45 |
| Isopropanol | 10,0 | 10,0 | 10,0 | 10,0 |
| Luft | 1,05 | - | - | - |
| Paridol^{R}P⁹ | - | - | - | 0,1 |

| | | | | |
|---|---|---|---|---|
| ⁸ davon 0,9 % Vergällungsmittel Diethylphthalat | | | | |
| ⁹ 4-Hydroxybenzoesäurepropylester (NAARDEN) | | | | |

## Patentansprüche

1. Zubereitung zum Festigen von Haaren auf Basis einer alkoholischen oder wäßrig-alkoholischen Lösung, enthaltend Filmbildner und Lösungsmittel sowie übliche kosmetische Bestandteile, dadurch gekennzeichnet, daß
- die Zubereitung zu mindestens 40 Gew.-% aus Ethanol besteht,
- frei ist von fluorchlorkohlenwasserstoffhaltigen Treibmitteln und Parfümölen,
- der Filmbildner ein anionisches, amphoteres oder zwitterionisches Polymeres oder Copolymeres ist, das als Monomeres eine ganz oder teilweise neutralisierte, ethylenisch ungesättigte Carbonsäure mit 3 oder 4 Kohlenstoffatomen oder eine ganz oder teilweise neutralisierte, ethylenisch ungesättigte Dicarbonsäure mit 4 Kohlenstoffatomen oder deren Monoester mit einem C₁₋₄-Alkohol enthält und frei ist von Vinylpyrrolidon-Gruppen und die Zubereitung Phthalsäureester in einer solchen Menge enthält, daß das Verhältnis der Massen von Filmbildner und Phthalsäureester 3:1 bis 10:1 beträgt.

2. Zubereitung nach Anspruch 1, dadurch gekennzeichnet, daß sie als Lösungsmittel 50 bis 95 Gew.-% niederen Alkohol und 0 bis 20 Gew.-% Wasser, jeweils bezogen auf die gesamte Zubereitung, enthält.

3. Zubereitung nach einem der Ansprüche 1 oder 2, dadurch gekennzeichnet, daß zur Neutralisierung des Filmbildners ein organisches Amin dient, das ausgewählt ist aus Triethanolamin und 2-Amino-2-methyl-1-propanol.

4. Zubereitung nach einem der Ansprüche 1 bis 3, dadurch gekennzeichnet, daß der Filmbildner ein anionisches Polymeres ist.

5. Zubereitung nach einem der Ansprüche 1 bis 4, dadurch gekennzeichnet, daß der Filmbildner in einer Menge von 1 bis 10 Gew.-%, bezogen auf die gesamte Zubereitung, enthalten ist.

6. Zubereitung nach einem der Ansprüche 1 bis 5, dadurch gekennzeichnet, daß sie frei von Treibmitteln ist.

7. Zubereitung nach einem der Ansprüche 1 bis 6, dadurch gekennzeichnet, daß sie frei von Konservierungsmitteln ist.

8. Zubereitung nach Anspruch 1 bis 7, dadurch gekennzeichnet, daß der Phthalsäureester Diethylphthalat ist.

9. Verfahren zur Behandlung von Haaren, dadurch gekennzeichnet, daß eine Zubereitung nach einem der Ansprüche 1 bis 8 verwendet wird.

10. Verfahren nach Anspruch 9, dadurch gekennzeichnet, daß die Zubereitung nachträglich mit einem Parfümöl versetzt wurde.

## Claims

1. A preparation for setting hair based on an alcoholic or aqueous/alcoholic solution containing film formers and solvents and typical cosmetic components, characterized in that
- at least 40% by weight of the preparation consists of ethanol,
- the preparation is free from fluorochlorocarbon-containing propellants and perfume oils,
- the film former is an anionic, amphoteric or zwitterionic polymer or copolymer which contains as monomer a completely or partly neutralized ethylenically unsaturated carboxylic acid containing 3 or 4 carbon atoms or a completely or partly neutralized ethylenically unsaturated dicarboxylic acid containing 4 carbon atoms or a monoester thereof with a C₁₋₄ alcohol and is free from vinyl pyrrolidone groups and the preparation contains phthalic acid esters in such a quantity that the ratio by weight of film former to phthalic acid ester is 3:1 to 10:1.

2. A preparation as claimed in claim 1, characterized in that it contains as solvent 50 to 95% by weight of lower alcohol and 0 to 20% by weight of water, based on the preparation as a whole.

3. A preparation as claimed in claim 1 or 2, characterized in that an organic amine selected from triethanolamine and 2-amino-2-methyl-1-propanol is used to neutralize the film former.

4. A preparation as claimed in any of claims 1 to 3, characterized in that the film former is an anionic polymer.

5. A preparation as claimed in any of claims 1 to 4, characterized in that the film former is present in a quantity of 1 to 10% by weight, based on the preparation as a whole.

6. A preparation as claimed in any of claims 1 to 5, characterized in that it is free from propellants.

7. A preparation as claimed in any of claims 1 to 6, characterized in that it is free from preservatives.

8. A preparation as claimed in any of claims 1 to 7, characterized in that the phthalic acid ester is diethyl phthalate.

9. A process for the treatment of hair, characterized in that the preparation claimed in any of claims 1 to 8 is used.

10. A process as claimed in claim 9, characterized in that a perfume oil is subsequently added to the preparation.

## Revendications

1. Préparation pour la fixation des cheveux, à base d'une solution alcoolique ou aqueuse-alcoolique, renfermant un agent filmogène et un solvant, ainsi que des constituant cosmétiques usuels, caractérisée en ce que
- la préparation se compose à au moins 40 % en poids d'éthanol,
- est exempte d'agents propulseurs contenant des chlorofluorocarbures et d'huiles ou d'essences parfumées et
- l'agent filmogène est un polymère ou un copolymère anionique, amphotère ou zwitterionique, qui contient comme monomère un acide carboxylique à insaturation éthylénique, comportant 3 ou 4 atomes de carbone, neutralisé totalement ou partiellement ou un acide dicarboxylique à insaturation éthylénique comportant 4 atomes de carbone, neutralisé totalement ou partiellement ou leur monoester avec un alcool en C₁₋₄, et est exempt de groupes vinylpyrrolidone, et en ce que la préparation contient des esters de l'acide phtalique dans une proportion telle que le rapport entre les masses d'agent filmogène et d'esters d'acide phtalique atteint 3:1 à 10:1.

2. Préparation selon la revendication 1, caractérisée en ce qu'elle renferme comme solvant 50 à 95 % en poids d'alcool inférieur et 0 à 20 % en poids d'eau, dans chaque cas par rapport à la totalité de la préparation.

3. Préparation selon une des revendications 1 à 2, caractérisée en ce qu'une amine organique, choisie entre la triéthanolamine et le 2-amino-2-méthyl-1-propanol, sert à la neutralisation de l'agent filmogène.

4. Préparation selon une des revendications 1 à 3, caractérisée en ce que l'agent filmogène est un polymère anionique.

5. Préparation selon une des revendications 1 à 4, caractérisée en ce que l'agent filmogène est contenu dans une proportion de 1 à 10 % en poids par rapport à la totalité de la préparation.

6. Préparation selon une des revendications 1 à 5, caractérisée en ce qu'elle est exempte d'agents propulseurs.

7. Préparation selon une des revendications 1 à 6, caractérisée en ce qu'elle est exempte d'agents conservateurs.

8. Préparation selon une des revendications 1 à 7, caractérise en ce que l'ester d'acide phtalique est le diéthylphtalate.

9. Procédé de traitement des cheveux, caractérisé en ce qu'une préparation selon une des revendications 1 à 8 est utilisée.

10. Procédé selon la revendication 9, caractérisé en ce que la préparation est additionnée postérieurement d'une huile ou d'une essence parfumée.
